# EUROPEAN PATENT SPECIFICATION

(11) **EP 3 912 623 B1**
(45) Date of publication and mention of the grant of the patent: **31.07.2024**
(21) Application number: 20175739.0
(22) Date of filing: 20.05.2020
(51) Int. Cl.: A61K 31/166, A61K 31/18, A61K 31/4184, A61K 31/44, A61K 31/4523, A61K 31/519

(54) **PD-0184264 FOR THE TREATMENT OF CORONAVIRUS INFECTIONS AND/OR COVID-19 CYTOKINE STORM**
PD-0184264 ZUR BEHANDLUNG VON CORONAVIRUS-INFEKTIONEN UND/ODER COVID-19-CYTOKIN-STURM
PD-0184264 POUR LE TRAITEMENT DES INFECTIONS À CORONAVIRUS ET/OU D'UNE TEMPÊTE DE CYTOKINES DE LA COVID-19

(43) Date of publication of application: 24.11.2021
(73) Proprietor: Atriva Therapeutics GmbH, 72072 Tübingen (DE)
(72) Inventor: LUDWIG, Stephan, 49161 Muenster (DE); PLANZ, Oliver, 72581 Dettingen an der Erms (DE); HOFFMANN, Helen Elisa, 72076 Tuebingen (DE); KOCH-HEIER, Julia, 72127 Kusterdingen (DE); SCHINDLER, Michael, 72770 Reutlingen (DE)
(74) Representative: Schiweck Weinzierl Koch Patentanwälte Partnerschaft mbB

(56) References cited:
- WO-A2-02/069960
- YINGYUN CAI ET AL: "Suppression of Coronavirus Replication by Inhibition of the MEK Signaling Pathway", JOURNAL OF VIROLOGY, vol. 81, no. 2, 1 November 2006 (2006-11-01), US, pages 446 - 456, XP055726893, ISSN: 0022-538X, DOI: 10.1128/JVI.01705-06
- JASON KINDRACHUK ET AL: "Antiviral Potential of ERK/MAPK and PI3K/AKT/mTOR Signaling Modulation for Middle East Respiratory Syndrome Coronavirus Infection as Identified by Temporal Kinome Analysis", ANTIMICROBIAL AGENTS AND CHEMOTHERAPY, vol. 59, no. 2, 1 February 2015 (2015-02-01), US, pages 1088 - 1099, XP055726785, ISSN: 0066-4804, DOI: 10.1128/AAC.03659-14
- TEIJARO JOHN R ED - EILAT DAN: "Cytokine storms in infectious diseases", SEMINARS IN IMMUNOPATHOLOGY, SPRINGER BERLIN / HEIDELBERG, DE, vol. 39, no. 5, 3 July 2017 (2017-07-03), pages 501 - 503, XP036269709, ISSN: 1863-2297, [retrieved on 20170703], DOI: 10.1007/S00281-017-0640-2
- HUANG CHAOLIN ET AL: "Clinical features of patients infected with 2019 novel coronavirus in Wuhan, China", THE LANCET, ELSEVIER, AMSTERDAM, NL, vol. 395, no. 10223, 24 January 2020 (2020-01-24), pages 497 - 506, XP086050317, ISSN: 0140-6736, [retrieved on 20200124], DOI: 10.1016/S0140-6736(20)30183-5

## Description

### FIELD OF THE INVENTION

The present invention relates to the use of the MEK-inhibitor PD-0184264 or a pharmaceutically acceptable salt thereoffor the treatment of coronavirus infections and/or the treatment or prevention of COVID-19 cytokine storm.

### BACKGROUND

The Coronavirus group consists of enveloped positive stranded RNA viruses belonging to the family *Coronaviridae* and comprise subtypes referred to as Alpha-, Beta-, Gamma- and Delta coronavirus. Alpha and Beta affect mammals, while Gamma affects birds and Delta can affect both. The coronavirus family comprises several well-known disease-causing members. The Betacoronavirus family, has so far posed the biggest risk to humans and now includes the most well-known virus targets including Severe Acute Respiratory Syndrome coronavirus (SARS-CoV-1) responsible for the deaths of 774 people in 2003, Middle East Respiratory Syndrome coronavirus (MERS-CoV), believed to of killed 858 people in 2012, and the newest form to emerge, the novel coronavirus, (SARS-CoV-2) which as of early May 2020 has caused over 250,000 deaths worldwide. In all, coronaviruses represent a continued and ever evolving threat to human life.

While the different subtypes of coronaviruses can infect different host types, the overall biochemical structure of the virus is similar. Despite the current attempts of developing vaccines against a human-pathogenic coronavirus infection, there remains a need to provide alternative or improved compositions and methods for treatment and/or prevention of diseases caused by human-pathogenic coronaviruses.

The COVID-19 pandemic in 2019/20 caused by SARS-CoV-2 clearly demonstrates that coronaviruses have a strong impact on global health systems. No preventative vaccination is available, and only one antiviral drug, Remdesivir, is provisionally approved for COVID-19. While various attempts have been made to treat COVID-19 with available drugs, so far none has been very successful. This highlights the urgent need for additional effective antivirals to better control the infections of coronaviruses in general and SARS-CoV-2 in particular. Notably, in the early phase of a pandemic, when no vaccine is available, antivirals are the stand-alone treatment.

In the case of COVID-19, the clinical spectrum of SARS-CoV-2 infection appears to be wide, encompassing asymptomatic infection, mild upper respiratory tract illness, and severe viral pneumonia with respiratory failure and even death, with many patients being hospitalized. The use of a 3-stage classification system is widely accepted, recognizing that COVID-19 illness exhibits three grades of increasing severity which correspond with distinct clinical findings, response to therapy and clinical outcome. Despite high numbers of infections, 80% of all positively tested patients experience mild symptoms only, 20% show signs of hypoxemia leading to hospitalization and only 5% require treatment in intensive care units (ICU). In order to treat COVID-19, it is important to understand the stages of the infection. These stages are summarized in Hasan et al., entitled "COVID-19 Illness in Native and Immunosuppressed States: A Clinical-Therapeutic Staging Proposal" and summarized below.

The initial stage, termed Stage I, is a mild infection and occurs at the time of inoculation and early establishment of disease. For most people, this involves an incubation period associated with mild and often non-specific symptoms for some days such as malaise, fever, and a dry cough. In patients who can keep the virus limited to this stage of COVID-19, prognosis and recovery is excellent. Treatment at this stage is primarily targeted towards symptomatic relief. Should an antiviral therapy be proven beneficial, targeting selected patients during this stage may reduce duration of symptoms, minimize contagiousness, and prevent progression of severity.

In the second stage, termed Stage II, of an established pulmonary disease, viral multiplication and localized inflammation in the lung is the norm. Stage II includes pulmonary involvement, termed Stage IIa, without and Stage IIb with hypoxia. During this stage, patients develop a viral pneumonia, with cough, fever and possibly hypoxia. Over the course of the disease, dyspnea occurs after a median of 13 days after the first onset of symptoms (range 9-16.5 days). Dyspnea is a sign of serious disease of the airway, lungs, or heart and is characterized by difficult or labored breathing and shortness of breath. In the case of COVID-19, imaging with chest X-ray or computerized tomography reveals bilateral infiltrates or ground glass opacities. It is at this stage that most patients with COVID-19 need to be hospitalized for close observation and management. Treatment primarily consists of supportive measures and antiviral therapies, once available. It is possible that patients will nevertheless progress to Stage III to require mechanical ventilation in an intensive care unit (ICU).

In Stage II COVID-19, markers of systemic inflammation may be elevated, but not remarkably so. In early studies performed on the first group of patients in Wuhan, China, it was found that upon entry into the hospital, plasma IL1beta, IL1Ralpha, IL7, IL8, IL9, IL10, basic FGF, GCSF, GMCSF, IFNgamma, IP10, MCP1, MIP1alpha, MIP1beta, PDGF, TNFalpha, and VEGF concentrations were higher than in healthy adults. In addition, it was found that patients in ICU had plasma concentrations of IL2, IL7, IL10, GCSF, IP10, MCP1, MIP1alpha, and TNFalpha were higher than in patients upon admission to the hospital (Huang et al.; The Lancet; Vol 395; pp.: 497-506 February 15, 2020), indicating that an increase in these cytokines marks the transition from COVID-19 stage II to stage III.

A minority of COVID-19 patients will transition into the third and most severe stage of illness, termed Stage III, which manifests as an extra-pulmonary systemic hyperinflammation syndrome. In this stage, markers of systemic inflammation are elevated. Overall, the prognosis and recovery from this critical stage of illness is poor.

Therefore, there is a need for compounds that are able to prevent progression of COVID-19 from Stage II to Stage III and reduce severity of the COVID-19 infection and mortality. Several multinational studies are ongoing focusing on compositions that have already been approved or undergone clinical studies for a different disease. Most of the compounds being considered for the treatment of COVID-19 belong to one of the two groups:
1. Antiviral medicines, that were originally developed against HIV, Ebola, Hepatitis C, or Influenza. The idea is that these block the reproduction of the virus or prevent entry into the lung cells. Among others, this includes old malaria medicines that are known to be effective against viruses. These antivirals may be effective in stage I and early stage II of Covid-19 as they act early on in the viral lifecycle.
2. Suppressive immunomodulators that were developed to treat rheumatoid arthritis or inflammatory bowel disease. The idea is that these would restrict the immune system so that these do not cause more damage than the virus itself. These could be effective in late Stage II and Stage III Covid-19 infection, as they suppress cytokine production.

Several multinational studies have been started to test the efficacy of Remdesivir (RNA polymerase inhibitor), Ritonavir or Lopinavir (HIV medication), Beta Interferons, and/or Chloroquine or Hydroxychloroquine (Malaria medication).

In early May, an emergency authorization was provided by the US FDA for the use of the antiviral Remdesivir in the treatment of hospitalized Covid-19 patients. Remdesivir is an RNA polymerase inhibitor that was originally developed for the treatment of Ebola, where it was found to be ineffective. Remdesivir was only approved to treat patients in a hospital setting showing severe symptoms which we would classify as stage III COVID-19. However, while Remdesivir was found to decrease the length of hospitalization of the patients in trials, there was no significant effect on mortality.

Cai et al. (Journal of Virology; Vol. 395; No. 2; pp.: 446-456 November 1, 2006) discloses that U0126 significantly impaired murine coronavirus mouse hepatitis virus (MHV) progeny production. It is taught that the Raf/MEK/ERK signalling pathway plays a role in coronavirus RNA synthesis. U0126 has a general inhibitory effect on MHV propagation, independent of cell type and virus strain. It is also disclosed that it would be appear that MEK1/2 inhibitors have a broad effect on propagations of viruses from various families (positive-strand and negative-strand RNA viruses, retroviruses, DNA viruses). Only *in vitro* data are shown.

Kindrachuck et al. (Antimicrobial Agents and Chemotherapy; Vol. 59; No. 2; pp.: 1088-1099 February 1, 2015) discloses that the MAPK/ERK pathway is specifically modulated in response to MERS-CoV infection *in vitro* throughout infection and may represent novel drug targets for therapeutic intervention. Selumetinib and Trametinib amongst other inhibitors demonstrated the strongest inhibitory activity *in vitro.* U0126 was also tested and showed inhibitory activity when administered pre-infection. Only *in vitro* data are shown.

In view of the prior art and the multitude of ongoing studies, it is clear that there is the need of new compounds and compositions effective in the treatment of diseases caused by Coronavirus infections, particular in respiratory tract diseases such as COVID-19. In particular, there is a need for medication to treat patients in Stage II and Stage III COVID-19, to prevent disease progression and to decrease mortality.

### SUMMARY OF THE INVENTION

The solution to the above problem provided herein is the provision of MEK inhibitors for the treatment of coronavirus infections. Specifically, MEK inhibitors were found to be useful in the treatment of Stage II COVID-19, as they both prevent viral exit from the host cells by blocking MEK receptors and decrease the immune responses that ultimately lead to Stage III of Covid-19. Due to this dual mechanism, MEK inhibitors, and specifically ATR-002, also termed PD-0184264, are particularly promising for the treatment of Stage II COVID-19. In addition, MEK inhibitors could be useful in preventing disease progression from Stage II to Stage III and in treating Stage III COVID-19. MEK inhibitors are already known originally as chemotherapeutics and are being developed by the inventors for use in treating or preventing viral infections, specifically influenzavirus and hantavirus. ATR-002, also termed PD-0184264, is a metabolite of CI-1040 described first in the context of chemotherapeutics in 2004 (Wabnitz et al.) that is being developed by the inventors for use in the treatment or prevention of viral diseases, such as coronavirus infection. ATR-002 has the chemical structure shown below:

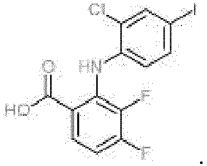

PD-0184264 is one of several metabolites of CI-1040 (Wabnitz et al., 2004, LoRusso et al., 2005). However, the mechanisms studied on PD-0184264 regarding the role of MEK in coronavirus infections apply equally to other MEK inhibitors.

For this reason, the present invention relates to the MEK inhibitor PD-0184264 or a pharmaceutically acceptable salt thereof for use in a method of treatment of a disease caused by a coronavirus in a human subject, wherein the human subject is hospitalized, wherein the coronavirus is SARS-CoV-2 and the patient is suffering from COVID-19. Also disclosed is an acute respiratory disease, such as those caused by SARS-CoV or MERS. In the context of the invention, the coronavirus is SARS-CoV2 and the corresponding patient to be treated is suffering from COVID-19. The use of the MEK inhibitor PD-0184264 of the invention is foreseen when the COVID-19 is Stage II COVID-19.

As mentioned above, the MEK inhibitor of the invention is PD-0184264 or pharmaceutically acceptable salt thereof. Also disclosed are the following MEK inhibitors CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof. Preferably, the MEK inhibitor is PD-0184264 or a pharmaceutically acceptable salt thereof.

In a preferred dosage form, the PD-0184264 or pharmaceutically acceptable salt thereof is administered to the human subject once daily in a dose of 100 to 1000 mg, preferably 300 to 900 mg, most preferably 300, 600 or 900 mg.

According to the invention, PD-0184264 is used to treat a hospitalized human patient suffering from COVID-19 caused by SARS-CoV-2. Preferably, the COVID-19 is Stage II COVID-19. The PD-0184264 can be administered to the human subject on 1 to 21 consecutive days, preferably 5 to 18 or 7 to 14 consecutive days after hospitalization. Preferably, the PD-0184264 is administered to the human subject in an oral dosage form.

In a further aspect, the MEK inhibitor of the invention PD-0184264 or a pharmaceutically acceptable salt thereof is used to treat a hospitalized human patient, wherein the coronavirus is resistant to previous antiviral treatment, such as Remdesivir. In addition, the MEK inhibitor PD-0184264 or a pharmaceutically acceptable salt thereof can be used to treat the hospitalized human subject that is over 60 years of age or belongs to a high risk group for coronavirus infection.

The invention is further directed to the use of the MEK inhibitor PD-0184264 or pharmaceutically acceptable salt thereof in treating or preventing a COVID-19 cytokine storm in a subject infected by a human coronavirus. The MEK inhibitor according to the invention is PD-0184264 or pharmaceutically acceptable salt thereof. Also disclosed are the following MEK inhibitors CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof.

The use of the MEK inhibitor PD-0184264 or pharmaceutically acceptable salt thereof in treating or preventing a COVID-19 cytokine storm in a subject may comprise reducing the level of IL-1β and/or TNF-α in the subject, preferably reducing the level of one of more, two or more, three or more, four or more, five or more or all six or TNF-a, IL-1ß, IP-10, IL-8, MCP-1, and MIP-1β in a subject.

In line with the above, the invention is also directed to the use of PD-0184264 or a pharmaceutically acceptable salt thereof in a method of treating or preventing a COVID-19 cytokine storm in a subject suffering from a human coronavirus infection, wherein the method comprises administering to the subject the MEK inhibitor PD-0184264 or pharmaceutically acceptable salt thereof. This use of PD-0184264 or pharmaceutically acceptable salt thereof in the method may comprise reducing the level of IL-1β and/or TNF-α in the (blood or plasma) of the subject, preferably reducing the level of one of more, two or more, three or more, four or more, five or more or all six or TNF- α, IL-1ß, IP-10, IL-8, MCP-1, and MIP-1β in (the blood or plasma) of the subject.

Finally, pharmaceutical compositions comprising the MEK inhibitor PD-0184264 or a pharmaceutically acceptable salt thereof for the use and medical treatments described herein are part of the invention.

### BRIEF DESCRIPTION OF THE FIGURES

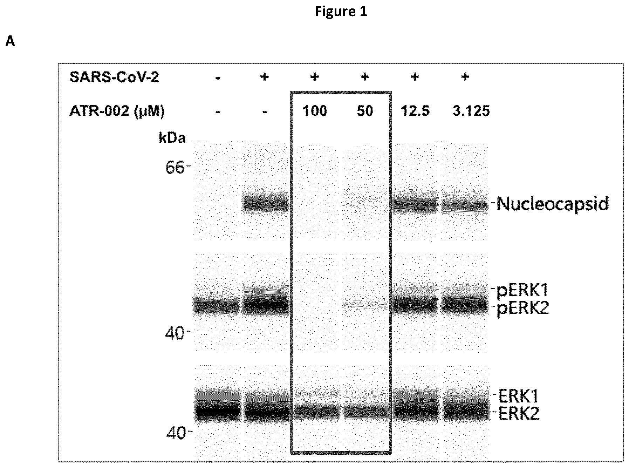

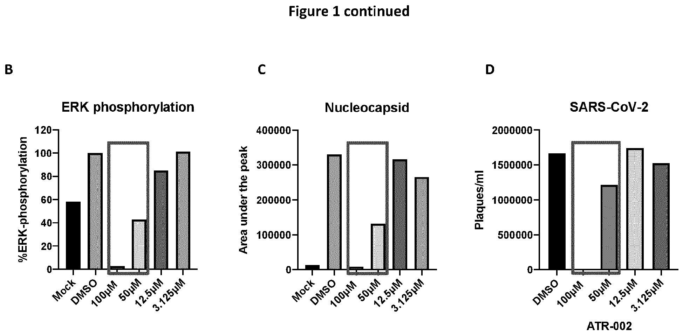

**Figure 1****:** **Fig. 1(A)** shows a lane view of Western Blot Analysis of SARS-CoV-2 infected and ATR-002 treated CaCo-2 cells
   pERK1/2, ERK1/2 and Nucleocapsid protein were detected in Caco2 cells infected with SARS-CoV2 treated with ATR-002. Uninfected cells (mock) were used as control.
**Fig.1 (B)****-(D)** show a comparison of ERK phosphorylation and correlation of Nucleocapsid protein of SARS-CoV-2 infected Caco-2 cells, treated with ATR-002
Fig. 1(B) shows ERK-phosphorylation. The area under the peak was used to normalize pERK1/2 to ERK1/2 and to calculate the values of %ERK-phosphorylation.
Fig. 1(C) shows nucleoside protein concentration of Caco-2 cells treated with different concentrations of ATR-002 compared to DMSO control.
Fig. 1(D) shows virus titer (PFU/mL) of Caco-2 cells treated with different concentrations of ATR-002 compared to DMSO control.
**Figure 2****:** **Fig.1 (A)** shows infection of Vero cells with SARS-CoV-2 upon MEK inhibition.
   **Fig.1 (B)** shows detection of the viral gene RdRP during infection with SARS-CoV-2 upon MEK inhibition.

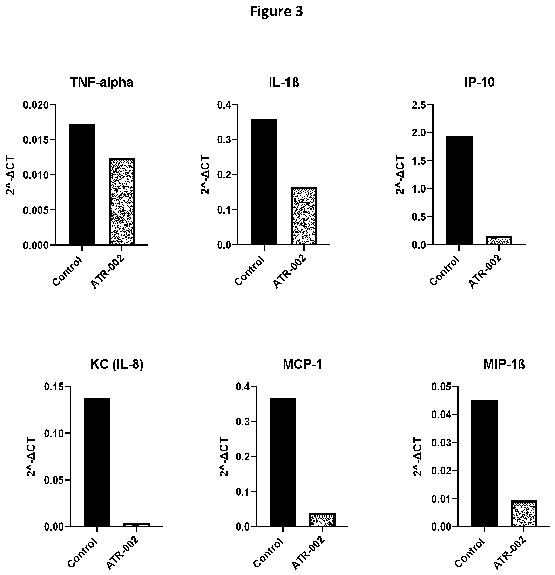

**Figure 3** shows that ATR-002 reduces cytokine & chemokine gene expression in an acute lung injury (ALI) mouse model.

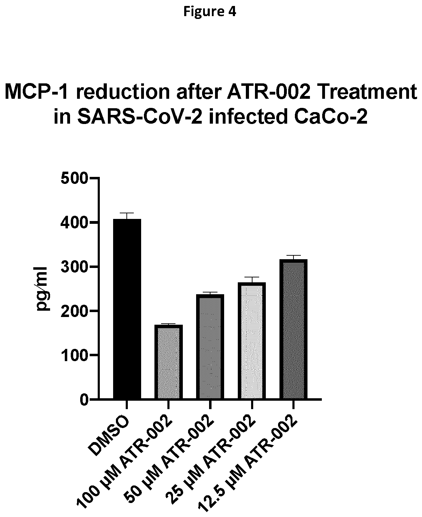

**Figure 4** shows that ATR-002 reduces the pro-inflammatory cytokine/chemokine response after SARS-CoV-2 infection of CaCo2 cells.

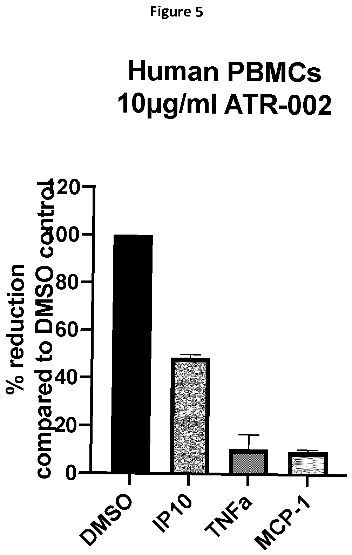

**Figure 5** shows that ATR-002 reduces the pro-inflammatory cytokine/chemokine response in PMBC cells.

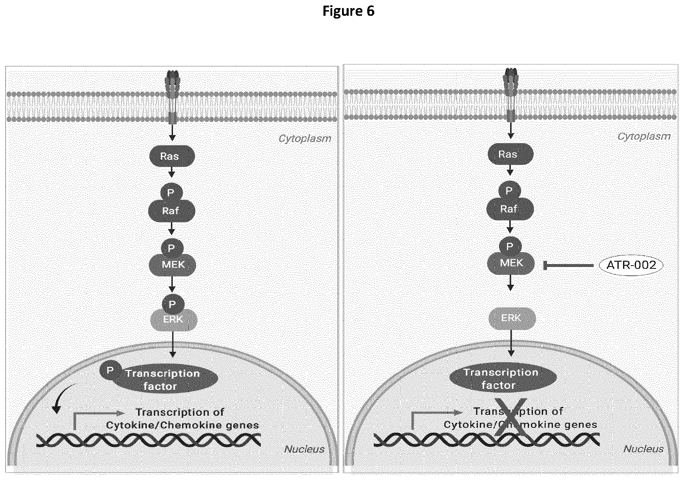

**Figure 6** depicts a schematic representation of modulation of the overwhelming cytokine response after ATR-002 treatment.

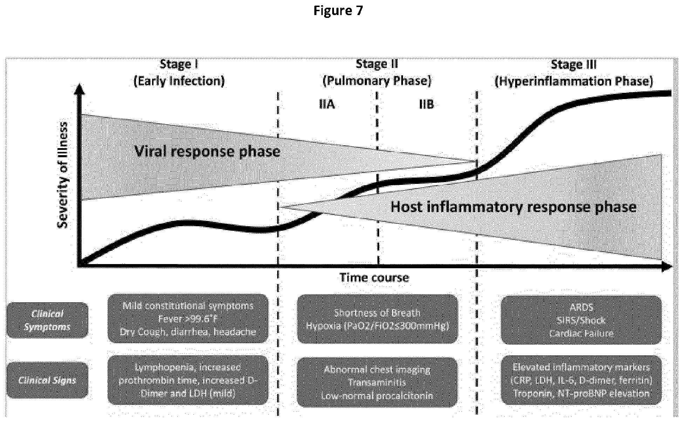

**Figure 7** represents the stages of COVID-19 according to Hasan et al.

### DETAILED DESCRIPTION

The following description includes information that may be useful in understanding the present invention. It is not an admission that any of the information provided herein is prior art or relevant to the presently claimed inventions, or that any publication specifically or implicitly referenced is prior art.

The above being said, the present invention relates to the MEK inhibitor PD-0184264 or a pharmaceutically acceptable salt thereof for use in a method of treatment of a viral disease in a hospitalized human patient caused by a coronavirus. As shown in the examples, the inventors showed that surprisingly the MEK inhibitor PD-0184264 or a pharmaceutically acceptable salt thereof has a dual effect in treating diseases such as COVID-19 caused by coronaviruses. The present invention also relates to the use of PD-0184264 or a pharmaceutically acceptable salt thereof in treating or preventing COVID-19 cytokine storm in patient being infected by a coronavirus, in particular being infected with SARS-CoV2.

According to the invention, as demonstrated in the appended Examples, ATR-002 shows a direct effect on viral propagation of SARS-CoV2 as well as an immunomodulatory effect leading to a decreased cytokine release. This dual effect is surprising and makes this MEK inhibitor particularly suited to the treatment of COVID-19 Stage II patients. As described in the background section above and defined further on, these patients are hospitalized by are not yet in intensive care. In these patients, MEK inhibition has the dual effect of preventing the cytokine storm that then leads to Stage III COVID-19 while simultaneously suppressing viral propagation to stop disease progression and decrease mortality.

In the first days after SARS-CoV-2 infection, extravasation of macrophages, neutrophils and NK cells into infected lung tissue is seen. COVID-19 is then caused by release of inflammatory mediators by infected lung epithelium, monocytes, dendritic cells and alveolar macrophages after infection with the SARS-CoV2 virus. The production and secretion of cytokines and chemokines (e.g. TNF-a, IL-1ß, IP-10, IL-8, MCP-1, MIP-1β) results in local and systemic acute inflammation and alterations of the adaptive immune response, which is termed the "COVID-19 cytokine storm" (which is also interchangeably referred to as "cytokine storm" herein) and marks the transition from Stage II to Stage III COVID-19. The term "COVID-19 cytokine storm is used herein within its regular meaning as used in the art (see, in this respect, Jamilloux et al, 2020) to mean a cytokine storm that may occur in a subject that has been infected with a human-pathogenic coronavirus, in particular SARS-CoV-2. From preliminary studies presented in the examples, it appears that MEK inhibitors in general and ATR-002 in particular would be able to simultaneously decrease the virus propagation and prevent the cytokine storm by suppressing cytokine release (cf. **Figure 6** in this respect). This dual effect was unexpected and is different from the other antivirals currently being studied for their useful in the treatment of COVID-19, such as Remdesivir.

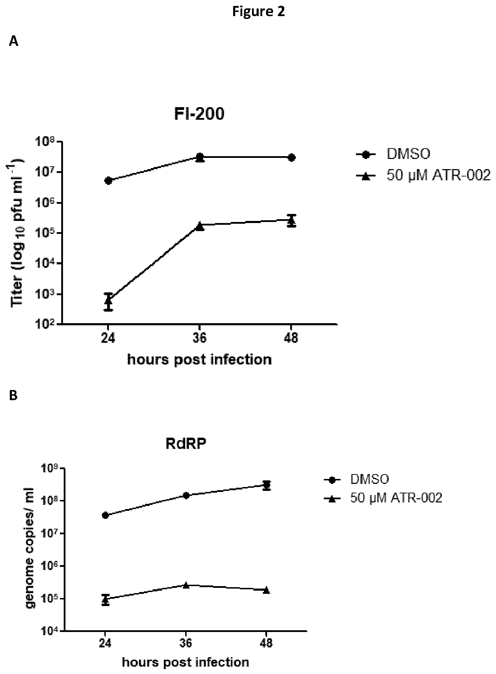

In light of the COVID-19 pandemic, the inventors were interested in determining whether SARS-CoV2 uses the MEK pathway for viral propagation. For this purpose, it was necessary to first obtain a SARS-CoV-2 isolate (Isolate "FI-100") and then determine which available cells could be infected with the virus. MEK inhibitors in general and ATR-002 in particular target the host cell factor MEK, the dose-response relationship of ATR-002 does not depend on the virus, but to the inhibition of the kinase. In *in vitro* experiments the antiviral efficacy of ATR-002 strongly depends on the activation status of MEK in the cell line used for the experiment. CaCo2 Cell-lines used for SARS-CoV-2 investigations below show a high constitutive MEK-activity. As a comparison, experiments in Vero cells were also performed. The specific methods used can be found in **Example 1** and the results are shown in **Figures 1** and **2****.** Specifically, Figure 1 shows that when CaCo-2 cells infected SARS-CoV-2 (MOI 0.1) were treated with different concentrations of ATR-002, inhibition of phosphorylation of ERK as well as SARS-CoV-2 and the nucleocapsid protein, which is a viral marker, was seen at concentrations of 50 and 100 µM ATR-002. This high amount of ATR-002 necessary for inhibition does not relate to the virus titer but is rather due to the choice of CaCo2 cells, which have constitutively active MEK, so that high amounts are necessary for inhibitor of MEK. These experiments show that SARS-CoV-2 uses the MEK pathway for virus export. Additionally, **Figure 2** shows that Vero cells infected with SARS-CoV-2 and treated with 50 µM ATR-002 have a lower viral load than control cells throughout the whole observation period and measurement of the SARS-CoV-2 viral gene RdRP confirmed this finding. This indicates that the inhibitor not just caused a delay in replication but sustainably inhibits viral propagation. From **Experiment 1**, the inventors concluded that SARS-CoV-2 uses the MEK pathway for viral propagation.

In a second step, the inventors reviewed data that had been gained previously in an acute lung injury (ALI) mouse model, which is presented as **Example 2.** Specifically, in the mouse ALI model, LPS induced cytokine & chemokine expression allows investigation of the immunomodulatory efficacy of ATR-002 in the absence of a virus infection. **Figure 3** shows that ATR-002 treatment of ALI mice leads to a decrease in the cytokines TNF-alpha, IL-1β, IP-10, IL-8, MCP-1, and MIP-1β. As discussed in the background section above, according to Huang et al., all of these cytokines are increased in COVID-19 patients, and TNF-alpha, IP-10 and MIP-1β are increased in Stage III COVID-19 patients as compared to the levels upon hospitalization with Stage II COVID-19. This indicates that, independent of SARS-CoV2 infection, ATR-002 is able to reduce cytokine and chemokine gene expression in mammals.

In order to confirm that ATR-002 reduces the pro-inflammatory cytokine/chemokine response after SARS-CoV-2 infection, further in vitro experiments were performed in CaCo2 cells as described in **Example 3.** CaCo2 cells were infected with SARS-CoV2 and treated with ATR-002. Amounts of MCP-1 were measured and results are shown in **Figure 4****.** Specifically, it was found that ATR-002 is able to reduce MCP-1 expression in SARS-CoV2 infected cells. However, a high amount of ATR-002 is necessary for this, as discussed in **Example 1,** this is due to the constitutive activation of the MEK pathway in CaCo2 cells. Due to somatic driver mutation, high amounts of ATR-002 are required to inhibit MCP-1 expression in SARS-CoV-2 infected CaCo-2 cells.

In order to see whether this effect is also seen in other cell types, the effect of MEK inhibition was studied in LPS induced Peripheral blood mononuclear cells (PMBC) as described in **Example 4.** Again, it was found that ATR-002 reduces the pro-inflammatory cytokine/chemokine response in PMBCs. Specifically, the amounts of IP10, TNF-alpha and MCP-1 expression were studied and as can be seen from **Figure 5**, all three were reduced after treatment with ATR-002, indicating that 10µg/ml ATR-002 is sufficient to inhibit >90% of LPS induced MCP-1 in human PBMCs.

Finally, due to the urgency of providing a treatment for the COVID-19 pandemic and in view of the promising data from **Examples 1-4** described herein as well as of positive data from a Phase I safety study of ATR-002, a Phase II study is planned. This phase II study is described in **Example 5.** The study is termed **RESPIRE** - A **R**andomized, Double-Blind, Placebo-Controlled, Clinical Study to **E**valuate the **S**afety and Efficacy of ATR-002 in Adult Hospitalized **P**atients with Moderate Coronavirus Disease (COVID-19). The primary objective of the study is to demonstrate the efficacy of ATR-002 compared to placebo, each on standard of care, in the treatment of patients with COVID-19 assessed by the clinical severity status at day 15. As determined by **Examples 1** to **4**, ATR-002 has the potential to treat COVID-19 due to its Mode of Action with a dual benefit (effect) being (1) an antiviral agent, and (2) immunomodulation (to interfere with the cytokine storm). This clinical study will enroll a total of 200 adult hospitalized patients suffering from moderate coronavirus disease Stage II. A nasopharyngeal swab will be taken immediately prior to randomization to confirm presence of SARS-CoV-2 thereafter.

All randomized patients will be receiving standard of care as per local standards. 100 Patients will be randomized to receive ATR-002 900 mg orally once daily, 100 patients will be receiving matching Placebo. ATR-002 or placebo will be supplied in a controlled double-blind fashion for 5 days.

The primary objective of the study is to evaluate the efficacy of ATR-002 relative to placebo measured by the clinical severity status on a 7-point ordinal scale [1] Not hospitalized, without limitations, [2] Not hospitalized, with limitations, [3] Hospitalized, not requiring supplemental oxygen, [4] Hospitalized, requiring supplemental oxygen, [5] Hospitalized, on non-invasive ventilation or high flow oxygen devices, [6] Hospitalized, on invasive mechanical ventilation or ECMO, [7] Death.

Secondary outcomes will be measured by clinical signs and symptoms, patient reported outcomes, Treatment Emergent Adverse Events, Serious Adverse Events, derived clinical parameters, scores and study events, changes in laboratory values and ATR-002 plasma levels, as well as changes in quantitative SARS-CoV-2 samples.

All patients will be followed up after end of study drug treatment until day 90 for safety reasons and to determine survival status.

The patients selected for the study are adults 18 years of age or older at screening and requiring hospitalization, showing clinical signs of a COVID-19 infection defined as having fever, defined as temperature ≥ 37.3 °C (axilla), ≥ 38.0 °C (oral), or ≥ 38.6 °C (rectal or tympanic) and SpO₂ <=94% on room air or requiring supplemental oxygen to maintain SpO₂>94%. Based on the antiviral and cytokine/chemokine studies shown in the Examples and further data from the Phase I clinical study, an inhibition of >50% - 90% of MEK activity is required to reduce viral load and cytokine/chemokine expression by more than 50%. In the intended RESPIRE study described above and in **Example 5**, patients suffering from Stage II COVID-19 will be treated.

As can be seen from **Figure 7** which is from a paper from Hasan et al., entitled "COVID-19 Illness in Native and Immunosuppressed States: A Clinical-Therapeutic Staging Proposal" and summarized below, in stage II COVID-19 both the viral response phase and the host response phase are ongoing, so that this would be the stage at which the use of a MEK inhibitor would be most useful. The stages of COVID-19 as determined by Hasan et al are summarized again below.

The initial stage, termed Stage I, is a mild infection and occurs at the time of inoculation and early establishment of disease. For most people, this involves an incubation period associated with mild and often non-specific symptoms for some days such as malaise, fever, and a dry cough. In patients who can keep the virus limited to this stage of COVID-19, prognosis and recovery is excellent. Treatment at this stage is primarily targeted towards symptomatic relief. Should an antiviral therapy be proven beneficial, targeting selected patients during this stage may reduce duration of symptoms, minimize contagiousness, and prevent progression of severity.

In the second stage, termed Stage II, of an established pulmonary disease, viral multiplication and localized inflammation in the lung is the norm. Stage II includes pulmonary involvement, termed Stage IIa, without and Stage IIb with hypoxia. During this stage, patients develop a viral pneumonia, with cough, fever and possibly hypoxia. Over the course of the disease, dyspnea occurs after a median of 13 days after the first onset of symptoms (range 9-16.5 days). Dyspnea is a sign of serious disease of the airway, lungs, or heart and is characterized by difficult or labored breathing and shortness of breath. In the case of COVID-19, imaging with chest X-ray or computerized tomography reveals bilateral infiltrates or ground glass opacities. It is at this stage that most patients with COVID-19 need to be hospitalized for close observation and management. Treatment primarily consists of supportive measures and antiviral therapies, once available. It is possible that patients will nevertheless progress to Stage III to require mechanical ventilation in an intensive care unit (ICU).

In Stage II COVID-19, markers of systemic inflammation may be elevated, but not remarkably so. In early studies performed on the first group of patients in Wuhan, China, it was found that upon entry into the hospital, plasma IL1β, IL1RA, IL7, IL8, IL9, IL10, basic FGF, GCSF, GMCSF, IFNy, IP10, MCP1, MIP1α, MIP1β, PDGF, TNFα, and VEGF concentrations were higher than in healthy adults. In addition, it was found that patients in ICU had plasma concentrations of IL2, IL7, IL10, GCSF, IP10, MCP1, MIP1α, and TNFα were higher than in patients upon admission to the hospital (Huang et al.; The Lancet; Vol 395; pp.: 497-506 February 15, 2020), indicating that an increase in these cytokines marks the transition from COVID-19 stage II to stage III. This transition marked by sudden and rapidly progressing clinical deterioration that is called the "COVID-19 cytokine storm" (also termed "cytokine storm" herein). As described in detail in Jamilloux et al. (Autoimmunity Reviews 2020), here markers of systemic inflammation are elevated, such as IL-1β, IL-Rα, IL-6, TNF-α and sIL2Rα. This corresponds to what was shown by Huang et al. discussed above.

A minority of COVID-19 patients will experience a COVID-19 cytokine storm and transition into the third and most severe stage of illness, termed Stage III, which manifests as an extra-pulmonary systemic hyperinflammation syndrome. Overall, the prognosis and recovery from this critical stage of illness is poor.

For this reason, the dual mechanism provided by MEK inhibition would be critical to prevent progressing into Stage III and therefore decrease mortality.

The MEK inhibitor of the present invention PD-0184264 or a pharmaceutically acceptable salt thereof is for use in a method for treating and/or prevention. As such the term "treating" or "treatment" includes administration of the MEK inhibitor PD-0184264 or a pharmaceutically acceptable salt thereof preferably in the form of a pharmaceutical composition, to a subject suffering from a coronavirus infection for the purpose of ameliorating or improving symptoms. Similarly included is the administration of the MEK inhibitor PD-0184264 or a pharmaceutically acceptable salt thereof preferably in the form of a pharmaceutical, to a subject suffering from a COVID-19 cytokine storm for the purpose of ameliorating or improving symptoms.

Furthermore, the term "prevent" as used herein, refers to a medical procedure whose purpose is to prevent a disease. As used herein, the terms "prevent", "prevention" and "preventing" refer to the reduction in the risk of acquiring or developing a given condition in a patient diagnosed with a coronavirus infection, such as a COVID-19 cytokine storm. Also meant by "prevention" is the reduction or inhibition of markers of systemic hyperinflammation, such as TNF-α, IL-1ß, IP-10, IL-8, MCP-1, and/or MIP-1β, in a subject diagnosed with a coronavirus infection, such as SARS-CoV-2 to reduce the risk of systemic hyperinflammation, such as a COVID-19 cytokine storm, in a subject.

"MEK inhibitors" inhibit the mitogenic signaling cascade Raf/MEK/ERK in cells or in a subject by inhibiting the MEK (mitogen-activated protein kinase kinase). This signaling cascade is hijacked by many viruses, in particular influenza viruses and corona viruses, to boost viral replication. Specific blockade of the Raf/MEK/ERK pathway at the bottleneck MEK therefore impairs growth of viruses, in particular corona viruses. Additionally, MEK inhibitors show low toxicity and little adverse side effects in humans. There is also no tendency to induce viral resistance (Ludwig, 2009). According to the invention, the MEK inhibitor is PD-0184264 or pharmaceutically acceptable salt thereof. Also disclosed are the following MEK inhibitors CI-1040, GSK-1120212, GDC-0973, Binimetinib, Selumetinib, PLX-4032, AZD6244, AZD8330, AS-703026, RDEA-119, RO-5126766, RO-4987655, PD-0325901, TAK-733, AS703026, PD98059 and PD184352 or pharmaceutically acceptable salt or metabolite thereof. The MEK inhibitor according to the invention is PD-0184264.

The "disease" caused by a corona virus may be an acute respiratory disease caused by SARS-CoV, SARS-CoV-2 or MERS. According to the invention, the coronavirus is SARS-CoV2 and the patient is suffering from COVID-19. In a most preferred version, the disease is Stage II COVID-19.

The "coronavirus" according to the invention is SARS-CoV2. Also disclosed are the following coronaviruses SARS-CoV or MERS or a related new zoonotic or mutant coronavirus. In one specific embodiment, the coronavirus is resistant to previous antiviral treatment, such as Remdesivir.

The "subject", which may be treated by the inhibitor, the MEK inhibitor of the present invention PD-0184264 or a pharmaceutically acceptable salt thereof, is a human subject that has been diagnosed with a coronavirus infection. In one embodiment, the subject is hospitalized. The subject may be of any age, and may be a child between 0 to 10 years, a teenager between 10 and 18 years or an adult of 18 years and above. The subject may optionally be between the ages of 50 and 65, between the ages of 18 or 50, or older than 65 years of age. In other embodiments the subject is selected from the group consisting of subjects who are at least 60 years old, subjects who reside in chronic care facilities, subjects who have chronic disorders of the pulmonary or cardiovascular system, subjects who required regular medical follow-up or hospitalization during the preceding year because of chronic metabolic diseases, renal dysfunction, hemoglobinopathies, or immunosuppression.

In one specific aspect, the subject may be treated with the MEK inhibitor PD-0184264 or a pharmaceutically acceptable salt thereof in order to prevent or treat a "COVID-19 cytokine storm". As mentioned above, this term is used within its regular meaning as used in the art (see, in this respect, Jamilloux et al, 2020) to mean a cytokine storm that may occur in subjects that have been infected with a human-pathogenic coronavirus, in particular SARS-CoV-2. Such a cytokine storm is marked by rapid clinical deterioration and an increase in pro-inflammatory cytokines marks the transition from Stage II to Stage III COVID-19. Specifically, both Huang et al. and Jamilloux et al. note that a sudden increase in IL-1β and/or TNF-α was observed, possibly together with an increase in two or more, three or more, four or more, five or more or all six of TNF-α, IL-1ß, IP-10, IL-8, MCP-1, and MIP-1β. In one aspect, the MEK inhibitor PD-0184264 or a pharmaceutically acceptable salt thereof is used to reduce the level of IL-1β and/or TNF-α in the subject, preferably reducing the level of one of more, two or more, three or more, four or more, five or more or all six of TNF-α, IL-1ß, IP-10, IL-8, MCP-1, and MIP-1β in a subject.

The inflammatory cytokines and chemokines cited above are well known in the art. Specifically, the terms TNF- α, IL-1ß, IP-10, IL-8, MCP-1, and MIP-1β refer to the human protein sequences known in UNIPROT and GENEBANK under the following accession numbers:

| Gene | GeneBank accession | Uniprot accession | species |
|---|---|---|---|
| TNF-α | NM_000594 | P01375 | |
| IL-1β | NM_000576 | P01584 | |
| IP-10 | NM_001565 | P02778 | Human |
| IL-8 | NM_000584 | P10145 | |
| MCP-1 | NM_002982 | P13500 | |
| MIP-1β | NM_002984 | P13236 | |

The pharmaceutical composition for the use of the invention and comprising the MEK inhibitor PD-0184264 is administered to a human patient that is hospitalized and is suffering from a disease caused by a coronavirus. In one aspect, the human patient is over 60 years of age or belongs to a high risk or very high risk group for corona virus infection. In the case of COVID-19, the very high risk group includes patients who:
- are over 70 years of age
- have had an organ transplant
- are undergoing active chemotherapy
- are having radical radiotherapy for lung cancer
- have cancers of the blood or bone marrow such as leukemia, lymphoma or myeloma who are at any stage of treatment
- are having immunotherapy or other continuing antibody treatments for cancer
- are having other targeted cancer treatments which can affect the immune system
- have had bone marrow or stem cell transplants in the last 6 months, or who are still taking immunosuppression drugs
- have severe respiratory conditions including cystic fibrosis, severe asthma, pulmonary fibrosis, lung fibrosis, interstitial lung disease and severe COPD
- have a condition that provide a very high risk of getting infections (such as SCID, homozygous sickle cell)
- are taking medicine that makes you much more likely to get infections (such as high doses of steroids or immunosuppression therapies)
- have a serious heart condition and are pregnant.

The high risk group includes people who:
- are over 60 years of age
- have a learning disability
- have a lung condition that's not severe (such as asthma, COPD, emphysema or bronchitis)
- have heart disease (such as heart failure)
- have high blood pressure (hypertension)
- have diabetes
- have chronic kidney disease
- have liver disease (such as hepatitis)
- have a medical condition that can affect your breathing
- have cancer
- have a weak immune system (immunosuppressed)
- have cerebrovascular disease
- have a condition affecting brain or nerves (such as Parkinson's disease, motor neuron disease, multiple sclerosis, or cerebral palsy)
- have a problem with their spleen or have the spleen removed
- have a condition that means you have a high risk of getting infections (such as HIV, lupus or scleroderma)
- are taking medicine that can affect the immune system (such as low doses of steroids)
- have obesity.

PD-0184264 or a pharmaceutically acceptable salt thereof for use in the method of the invention, PD-0184264 may be administered orally, intravenously, intrapleurally, intramuscularly, topically or via inhalation. Preferably, PD-0184264 is administered via inhalation or orally. In preferred embodiment, PD-0184264 is administered once daily in an oral dosage between 100mg and 1000mg, preferably 300mg, 600mg or 900mg, for on 1 to 21 consecutive days, preferably 5 to 18 or 7 to 14 consecutive days after hospitalization.

Specifically, as described previously, in Phase I clinical studies PD-0184264 was administered with a starting dose of 100 mg and up to three escalation steps in seven arms, following a single ascending dose/ multiple ascending dose (SAD / MAD) regime. Administration scheme was one dose PD-0184264, escalating 100 mg to 900 mg (SAD), followed by seven doses PD-0184264, escalating 100 mg to 600 mg QD over seven days (MAD). Each dose cohort was considered to be safe by the Safety Review Committee (SRC), with release allowed for the next higher dose (up to SAD 900 mg and MAD 600 mg, respectively). The observed pharmacokinetic profile supports the intended once-daily regime for the further clinical development, and a dosage of 900mg is intended for testing.

Only few adverse events in total and no serious adverse events were observed during the trial. PD-0184264 is thus considered to be safe and well tolerable. Pharmacokinetics exposure and determination of MEK inhibition were verified in the Phase I study and confirm the maintenance of clinically relevant blood levels.

The present invention also envisages different compositions, preferably pharmaceutical compositions. The present invention relates to a composition comprising PD-0184264 for use in a method for the treatment of a disease caused by a coronavirus, wherein the coronavirus is SARS-CoV-2 and the patient is suffering from COVID-19.

As mentioned above, the composition comprising the MEK inhibitor PD-0184264 or a pharmaceutically acceptable salt thereof may be a pharmaceutical composition. According to the invention, the pharmaceutical composition comprises PD-0184264. Preferably, such compositions further comprise a carrier, preferably a pharmaceutically acceptable carrier. The composition can be in the form of orally administrable suspensions or tablets, nasal sprays, preparations for inhalation devices, sterile injectable preparations (intravenously, intrapleurally, intramuscularly), for example, as sterile injectable aqueous or oleaginous suspensions or suppositories.

The MEK inhibitor PD-0184264 or a pharmaceutically acceptable salt thereof is preferably administered in a therapeutically effective amount. The "therapeutically effective amount" for PD-0184264 or each active compound/inhibitor can vary with factors including but not limited to the activity of the compound used, stability of the active compound in the patient's body, the severity of the conditions to be alleviated, the total weight of the patient treated, the route of administration, the ease of absorption, distribution, and excretion of the compound by the body, the age and sensitivity of the patient to be treated, adverse events, and the like, as will be apparent to a skilled artisan. The amount of administration can be adjusted as the various factors change over time.

The inhibitors, methods and uses described herein are applicable to human therapy. The compound described herein, PD-0184264, may be administered in a physiologically acceptable carrier to a subject, as described herein. Depending upon the manner of introduction, the compounds may be formulated in a variety of ways as discussed below. The concentration of therapeutically active compound in the formulation may vary from about 0.1-100 wt. %. The agents may be administered alone or in combination with other treatments. For example, for the treatment of Stage II COVID-19, the PD-0184264 may be administered at a dosage in the range of 10 to 100 mg/kg PD-0184264, preferably in the range of 25 to 75 mg/kg PD-0184264. Preferred administration is as a once daily dosage between 100 and 1000 mg, including any dosage amount in between such as 200, 300, 400, 500, 600, 700, 800, and 900 mg. In a preferred embodiment, administration is once daily and the dosage is 600mg or 900mg administered orally. PD-0184264 can be administered for a time period of 1 to 21 consecutive days or more, preferably for 5 to 18 and most preferably for 7 to 14 days after hospitalization.

The pharmaceutical compounds in the method of the present invention can be administered in any suitable unit dosage form. Suitable oral formulations can be in the form of tablets, capsules, suspension, syrup, chewing gum, wafer, elixir, and the like. Pharmaceutically acceptable carriers such as binders, excipients, lubricants, and sweetening or flavoring agents can be included in the oral pharmaceutical compositions. If desired, conventional agents for modifying tastes, colors, and shapes of the special forms can also be included.

For injectable formulations, the pharmaceutical compositions can be in lyophilized powder in admixture with suitable excipients in a suitable vial or tube. Before use in the clinic, the drugs may be reconstituted by dissolving the lyophilized powder in a suitable solvent system for form a composition suitable for intravenous or intramuscular injection.

In one embodiment, the reduction of the viral infection is a reduction in plaque forming units (PFU)/ml. The "plaque forming units" is a measure of the number of particles capable of forming plaques per unit volume, such as virus particles. It is a functional measurement rather than a measurement of the absolute quantity of particles: viral particles that are defective or which fail to infect their target cell will not produce a plaque and thus will not be counted. For example, a solution of coronavirus with a concentration of 1,000 PFU/µl indicates that 1 µl of the solution carries enough virus particles to produce 1000 infectious plaques in a cell monolayer. In the case of the present invention, a cell culture treated with an inhibitor shows a reduced number of plaque forming units in a culture after the treatment, when compared to a culture before the treatment with the MEK inhibitor PD-0184264.

For the purpose of the invention the active compound as defined above also includes the pharmaceutically acceptable salt(s) thereof. The phrase "pharmaceutically acceptable salt(s)", as used herein, means those salts of compounds of the invention that are safe and effective for the desired administration form. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylaminoethanol, histidine, procaine, etc.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

### EXAMPLES

The following examples illustrate the invention. These examples should not be construed as to limit the scope of the invention. The examples are included for purposes of illustration and the present invention is limited only by the claims.

### MATERIALS:

### 1. CELL LINES

| **Description** | **Species** | **Origin** | **Source** |
|---|---|---|---|
| Caco-2 | Homo sapiens | Epithelial cells, | University Hospital Tübingen |
| | | Colon, | Institute of Medical Virology and Epidemiology |
| | | Colorectal adenocarcinoma | |
| Vero E6 | *Cercopithecus aethiops,* Kidney | Epithelial cells, Kidney, normal | ATCC (American Type Culture Collection) Catalog. Nr. CRL-1586 |

### 2. VIRUS

| **Description** | **Species** | **Source** |
|---|---|---|
| SARS-CoV-2 | Severe acute respiratory syndrome coronavirus | Isolate "FI-100" |
| | | Westfaelische Wilhelms-University Muenster |
| | | Institute of Virology (IVM) |

### 3. DRUG

ATR-002 (PD0184264) [2-(2-chloro-4-iodophenylamino)-N-3,4-difluoro benzoic acid], (M = 409,55 g/mol) was synthesized at ChemCon GmbH (Freiburg, Germany). For all cell culture experiments, a 10 mM stock solution of ATR-002 was prepared in DMSO (Merck-Millipore, Darmstadt, Germany) and further diluted in the respective media.

### Example 1: SARS-CoV-2 uses the MEK pathway

In this initial experiment, the inventors were interested in finding out whether inhibition of the MEK pathway leads to inhibition of viral propagation. ATR-002 targets the host cell factor MEK, however the dose-response relationship of ATR-002 does not relate to the virus, but to the inhibition of the kinase. In *in vitro* experiments the antiviral efficacy of ATR-002 strongly depends on the activation status of MEK in the cell line used for the experiment. CaCo2 Cell-lines used for SARS-CoV-2 investigations below show a high constitutive MEK-activity. As a comparison, experiments in Vero cells were also performed.

### Methods:

### A. Virus Yield Reduction Assay (VYR)

For the Virus yield reduction assay a 24-well plate (Greiner Bio-One, Cat. 662-160) with 95% confluent Caco-2 cells was prepared. The wells were washed once with Caco-2 infection medium DMEM (Gibco, Cat. 41965-039) supplemented with 5% FCS (Capricorn, Cat. FBS-12A), 1% Penicillin/Streptomycin, (Sigma Aldrich, Cat. P4333) and 1% Non-essential amino acids (NEAA) (Merck, Cat. K 0293). Cells were infected with SARS-CoV-2 (MOI 0.1) for 1h in 200µL Caco-2 infection medium per well. Afterwards the Virus inoculum was removed completely and cells were treated with 1mL per well with different concentrations of ATR-002 (100µM, 50µM, 25µM, 12.5µM, 6.25µM, 3.125µM, 1.56µM, 0.78µM, 0.39µM, 0.195µM, 0.098µM, 0µM) in Caco-2 infection medium with 1% DMSO for 24h at 37°C, 5% CO₂. Supernatants were harvested 24h post infection and centrifuged at max. speed, 5min, 4°C to remove cell debris. Aliquots à 200µL were prepared and stored at -80°C. The cell layer of each well was lysed by addition of 50µL modified RIPA Buffer supplemented with phosphatase and protease inhibitor cocktails, incubation 15min, 4°C. Lysates were centrifuged at max. speed, 5min, 4°C, to remove cell debris. Lysates were stored at -80°C.

### B. Plaque Assay Infection of Vero cells with SARS-CoV-2 upon MEK inhibition

To determine the virus titer a standard plaque assay was performed. Vero cells were infected with SARS-CoV-2 strain FI-200 (MOI 0.01). 1 hour post infection, the cells were treated with 50 µM ATR-002 or the respective amount of DMSO as a control. The compounds were present in the culture medium during the complete infection time. After 24, 36 and 48 hpi, supernatants were collected and virus titers quantified by plaque assay below. Data of a single experiment with three biological replicates are shown in **Figure 2A****.**

Vero E6 cells were seeded at 100% confluency in 6-well plates (Greiner Bio-One, Cat. 657-160). 6-fold dilutions of virus samples were prepared in Vero E6 infection medium (IMDM, Gibco, Cat. 12440-053) supplemented with 5% FCS (Capricorn, Cat. FBS-12A) and 1% Penicillin/Streptomycin (Sigma Aldrich Cat. P4333). The cells were washed once with Vero E6 infection medium before infection with 1mL per well of the virus dilutions for 1h at 37°C, 5% CO₂. Afterwards the Virus inoculum was removed completely, and the cells were overlaid with Avicel-Medium (1,25% Avicel (FMC BioPolymer, Cat. RC581), 10% MEM (Gibco, Cat. 21430-20),0,01% DEAE-Dextran (Sigma Aldrich, Cat.No.: D9885), 2,8% NaHCO₃ (Merck, Cat. No: 1.06329.1000), 1% Penicillin/Streptomycin (Sigma Aldrich, Cat. P4333), 0,2% BSA (Carl Roth, Cat. 9163.4), 1% L-Glutamine (Sigma Aldrich, Cat. G7513). Incubation at 37°C, 5% CO₂. After 72h the Avicel-Medium was removed. The cells were rinsed twice with PBS (Gibco, Cat. 14190-094), fixed with 4% Roti-Histofix (Carl Roth, Cat. A146.1) in PBS (Lonza, Cat. 17515Q) for 30min at 4°C and stained with Crystal violet solution (1% Crystal violet (Merck, Cat. 1408), 10% Ethanol (SAV-IP, Cat. ETO-5000-99-1) in ddH₂O). The virus titer was calculated based on the number of plaques per well and the respective dilution factor.

**C. Detection of the viral gene RdRP during infection with SARS-CoV-2 upon MEK inhibition.** Vero cells were infected with FI-200 (MOI 0.01). 1 hour post infection, the cells were treated with 50 µM ATR-002 or the respective amount of DMSO as a control. The compounds were present in the culture medium during the complete infection time. After 24, 36 and 48 hpi, supernatants were collected and used for RNA extraction. Extrapolated genome copy number per ml of the RdRP gene based on a standard curve after 24, 36 and 48 hpi. Data of a single experiment with three biological replicates are shown in **Figure 2B****.**

### D. Wes analysis

Wes^{™} capillary electrophoresis by ProteinSimple^{®} was used to identify and quantify pERK1/2, ERK1/2 and SARS-CoV-2 Nucleoprotein. Cell lysates were diluted with 0.1X Sample Buffer (ProteinSimple, Abingdon, Oxford, UK) to approximately 50ng total protein per lane and analyzed using specific antibodies. The primary antibodies pERK1/2 (Phospho-p44/42 MAPK (Erk1/2) (Thr202/Tyr204) (D13.14.4E) XP^{®} Rabbit mAb, Cat. 43702) and ERK1/2 (p44/42 MAPK (Erk1/2) (137F5) Rabbit mAb, Cat. 4695) were purchased from Cell Signaling Technology (Cell Signaling Technology, Danvers, Massachusetts, U.S.A.) and used at 1:50 (for pERK1/2) and 1:100 (ERK1/2) dilutions in antibody diluent (ProteinSimple, Abingdon, Oxford, UK). The SARS-CoV-2 Nucleoprotein Antibody, monoclonal mouse Ab was purchased from ProSci (Cat. 35.580) and used at 1µg/ml in antibody diluent (ProteinSimple, Abingdon, Oxford, UK). The anti-rabbit secondary antibody (ProteinSimple, Cat. DM-001) and anti-mouse secondary antibody (ProteinSimple, Cat. DM-002) and all other reagents for WES analysis were also purchased from ProteinSimple and were ready to use.

### Results:

The results of the above methods can be seen in **Figures 1** and **2****.** **Figure 1** shows that when CaCo-2 cells infected SARS-CoV-2 (MOI 0.1) were treated with different concentrations of ATR-002, inhibition of ERK as well as SARS-CoV-2 and the nucleocapsid protein, which is a viral marker, was seen at concentrations of 50 and 100 µM ATR-002. This high amount of ATR-002 necessary for inhibition does not relate to the virus titer but is rather due to the choice of CaCo2 cells, which have constitutively active MEK, so that high amounts are necessary for inhibitor of MEK. These experiments show that SARS-CoV2 uses the MEK pathway for virus export.

Additionally **Figure 2** shows that Vero cells infected with SARS-CoV-2 and treated with 50 µM ATR-002 have a lower viral load than control cells throughout the whole observation period of 24h, 36h and 48h, indicating that the inhibitor not just caused a delay in virus replication but inhibited propagation in a sustained manner. This was confirmed by measurement of the SARS-CoV2 viral gene RdRP.

### Example 2: ATR-002 reduces cytokine & chemokine gene expression in an acute lung injury (ALI) mouse model

The ALI mouse model of LPS induced cytokine & chemokine expression allows to investigate the immunomodulatory efficacy of ATR-002 in the absence of a virus infection.

### Methods:

Mice were anesthetized using ketamine (10 mg/kg) solution injected i.p. Both treatment groups were stimulated with 5mg/kg LPS prepared in PBS via i.p route. One hour post stimulation the treatment group was treated with 25 mg/kg ATR-002 via i.p route. Mice were euthanized 6h post treatment and lungs were preserved directly in RNAlater. RNA was isolated from lungs using RNeasy plus universal Midi kit (Qiagen, Hilden, Germany) according to the manufacturer instructions. RNA elution was perform in two rounds with RNase-free water and stored at -20 °C for further analysis. The RNA quality was determined using a NanoDrop spectrophotometer (ThermoFisher scientific) and was reverse transcribed using a RT² First Strand Kit (Qiagen, Hilden, Germany). Afterwards, the cDNA was used on the real-time RT² Profiler PCR Array in combination with RT² SYBR^{®} Green qPCR Mastermix. For optimal performance, the arrays was customized to include: 84 genes and 5 house-keeping genes to be used for data normalization. For quality control, mouse genomic DNA contamination test, 3 reverse transcription efficiency tests and 3 PCR array reproducibility test were included. Results for expression of genes coding for cytokines and chemokines involved in moderate and severe COVID-19 are presented.

The following sequences were used:

| Gene | GeneBank accession | Uniprot accession | species |
|---|---|---|---|
| TNF | NM_013693 | P06804 | |
| IL-1b | NM_008361 | P10749 | |
| IP-10 | NM_021274 | P17515 | |
| CXCL1 | | | Mouse |
| (KC) | NM_008176 | P12850 | |
| MCP-1 | NM_011333 | P10148 | |
| MIP-1β | NM_013652 | P14097 | |

### Results:

Figure 3 shows that ATR-002 treatment of ALI mice leads to a decrease in the cytokines TNF-alpha, IL-1beta, IP-10, IL-8, MCP-1, and MIP-1beta. As discussed in the background section above, according to Huang et al., all of these cytokines are increased in COVID-19 patients, and TNF-alpha, IP-10 and MIP-1beta are increased in Stage III COVID-19 patients as compared to the levels upon hospitalization with Stage II COVID-19. This indicates that, independent of SARS-CoV-2 infection, ATR-002 is able to reduce cytokine and chemokine gene expression in mammals.

### Example 3: ATR-002 reduces the pro-inflammatory cytokine/chemokine response after SARS-CoV-2 infection in CaCo2 cells

CaCo2 cells were infected with SARS-CoV-2 and treated with ATR-002 as described in Example 1. Amounts of MCP-1 were measured and results are shown in **Figure 4****.** Specifically, ATR-002 is able to reduce MCP-1 expression in SARS-CoV-2 infected cells. However, a high amount of ATR-002 is necessary for this, as discussed in **Example 1,** this is due to the constitutive activation of the MEK pathway in CaCo2 cells. Due to somatic driver mutation, high amounts of ATR-002 are required to inhibit MCP-1 expression in SARS-CoV-2 infected CaCo2 cells.

### Example 4: ATR-002 reduces the pro-inflammatory cytokine/chemokine response in Peripheral Blood Mononuclear cells (PMBC)

In order to check what concentration of ATR-002 would be necessary for cytokine reduction, the amounts of IP10, TNF-alpha and MCP-1 expression was studied in LPS induced PMBC cells after ATR-002 treatment. As can be seen from Figure 5, all three were reduced after treatment of 10µg/ml of ATR-002, indicating that 10µg/ml ATR-002 is sufficient to inhibit >90% of LPS induced MCP-1 in human PBMCs.

**Example 5: RESPIRE** - A **R**andomized, Double-Blind, Placebo-Controlled, Clinical Study to **E**valuate the Safety and Efficacy of ATR-002 in Adult Hospitalized **P**atients with Moderate Coronavirus Disease (COVID-19). The primary objective of the study is to demonstrate the efficacy of ATR-002 compared to placebo, each on standard of care, in the treatment of patients with COVID-19 assessed by the clinical severity status at day 15. ATR-002 has the potential to treat COVID-19 due to its Mode of Action with a dual benefit (effect) being (1) an antiviral agent, and (2) immunomodulation (to interfere with the cytokine storm). This clinical study will enroll a total of 200 adult hospitalized patients suffering from moderate coronavirus disease Stage II. A nasopharyngeal swab will be taken immediately prior to randomization to confirm presence of SARS-CoV-2 thereafter.

All randomized patients will be receiving standard of care as per local standards. 100 Patients will be randomized to receive ATR-002 900 mg orally once daily, 100 patients will be receiving matching Placebo. ATR-002 or placebo will be supplied in a controlled double-blind fashion for 5 days.

The primary objective of the study is to evaluate the efficacy of ATR-002 relative to placebo measured by the clinical severity status on a 7-point ordinal scale [1] Not hospitalized, without limitations, [2] Not hospitalized, with limitations, [3] Hospitalized, not requiring supplemental oxygen, [4] Hospitalized, requiring supplemental oxygen, [5] Hospitalized, on non-invasive ventilation or high flow oxygen devices, [6] Hospitalized, on invasive mechanical ventilation or ECMO, [7] Death. Secondary outcomes will be measured by clinical signs and symptoms, patient reported outcomes, Treatment Emergent Adverse Events, Serious Adverse Events, derived clinical parameters, scores and study events, changes in laboratory values and ATR-002 plasma levels, as well as changes in quantitative SARS-CoV-2 samples.

All patients will be followed up after end of study drug treatment until day 90 for safety reasons and to determine survival status.

The patients selected for the study are adults 18 years of age or older at screening and requiring hospitalization, showing clinical signs of a COVID-19 infection defined as having fever, defined as temperature ≥ 37.3 °C (axilla), ≥ 38.0 °C (oral), or ≥ 38.6 °C (rectal or tympanic) and SpO₂ <=94% on room air or requiring supplemental oxygen to maintain SpO₂>94%.

### REFERENCES

Haasbach, E. et al (2017). The MEK-inhibitor CI-1040 displays a broad anti-influenza virus activity in vitro and provides a prolonged treatment window compared to standard of care in vivo. Antiviral research 142, 178-184.
Hasan et al (2020). COVID-19 Illness in Native and Immunosuppressed States: A Clinical-Therapeutic Staging Proposal. J Heart Lung Transplant. 2020 Mar 20
Huang et al.; The Lancet; Vol 395; pp.: 497-506 February 15, 2020
Jamilloux et al.; Should we stimulate or suppress immune response in COVID-19? Cytokine and anti-cytokine interventions, Autoimmunity Reviews, AUTREV 102567 accepted 28 April 2020.
LoRusso, P., Adjei, A., Varterasian, M., Gadgeel, S., Reid, J., Mitchell, D., et al. (2005). Phase I and Pharmacodynamic Study of the Oral MEK Inhibitor CI-1040 in Patients With Advanced Malignancies. Journal of Clinical Oncology 23(23), 5281-5293.
Ludwig, S. (2009). Targeting cell signaling pathways to fight the flu: towards a paradigm change in anti-influenza therapy. Journal of Antimicrobial Chemotherapy, 64, 1-4.
Mackey, T.K., Liang, B.A., 2012. Lessons from SARS and H1N1/A: employing a WHO-WTO forum to promote optimal economic-public health pandemic response. J Public Health Policy 33, 119-130.
Pleschka, S., Wolff, T., Ehrhardt, C., Hobom, G., Planz, O., Rapp, U.R., Ludwig, S., 2001. Influenza virus propagation is impaired by inhibition of the Raf/MEK/ERK signalling cascade. Nat Cell Biol 3, 301-305. Wabnitz, A., Mitchell, D, and Wabnitz, D. (2004). In Vitro and in Vivo Metabolism of the Anti-Cancer Agent CI-1040, a MEK Inhibitor, in Rat, Monkey, and Human. Pharmaceutical Research 21(9), 1670-1679.

## Claims

1. PD-0184264 or a pharmaceutically acceptable salt thereof for use in a method of treatment of a disease caused by a coronavirus in a human subject, wherein the human subject is hospitalized, wherein the coronavirus is SARS-CoV-2 and the patient is suffering from COVID-19.

2. PD-0184264 for the use of claim 1, wherein the COVID-19 is Stage II COVID-19.

3. PD-0184264 for the use of claim 1 or 2, wherein PD-0184264 or pharmaceutically acceptable salt thereof is administered to a human subject once daily in a dose of 100 to 1000 mg, preferably 300 to 900 mg, most preferably 300, 600 or 900 mg.

4. PD-0184264 for the use of any one of claims 1-3, wherein PD-0184264 is administered to a human subject on 1 to 21 consecutive days, preferably 5 to 18 or 7 to 14 consecutive days after hospitalization.

5. PD-0184264 for the use of any one of claims 1 to 4, wherein PD-0184264 is administered to the human subject in an oral dosage form.

6. PD-0184264 for the use of any one of claims 1 to 5, wherein the coronavirus is resistant to previous antiviral treatment, wherein the previous antiviral treatment is preferably administration of Remdesivir.

7. PD-0184264 for the use of any one of claims 1 to 6, wherein the human subject that is hospitalized is over 60 years of age or belongs to a very high risk or a high risk group for the coronavirus.

8. PD-0184264 or pharmaceutically acceptable salt thereof for use in treating or preventing a COVID-19 cytokine storm in a subject infected by a human coronavirus.

9. PD-0184264 for the use of claim 8, wherein the use comprises reducing the level of IL-1β and/or TNF-α in the subject, preferably reducing the level of one of more, two or more, three or more, four or more, five or more or all six of TNF-α, IL-1ß, IP-10, IL-8, MCP-1, and MIP-1β in a subject.

10. A pharmaceutical composition comprising PD-0184264 or a pharmaceutically acceptable salt thereof for the use of any one of claims 1 to 9.

## Patentansprüche

1. PD-0184264 oder ein pharmazeutisch akzeptables Salz davon zur Verwendung in einem Verfahren zur Behandlung einer durch ein Coronavirus verursachten Krankheit bei einem menschlichen Subjekt, wobei das menschliche Subjekt stationär im Krankenhaus ist, wobei das Coronavirus SARS-CoV-2 ist und der Patient an COVID-19 leidet.

2. PD-0184264 für die Verwendung nach Anspruch 1, wobei COVID-19 COVID-19 der Stufe II ist.

3. PD-0184264 für die Verwendung nach Anspruch 1 oder 2, wobei PD-0184264 oder pharmazeutisch akzeptables Salz davon einem menschlichen Subjekt einmal täglich in einer Dosis von 100 bis 1000 mg, vorzugsweise 300 bis 900 mg, am meisten bevorzugt 300, 600 oder 900 mg verabreicht wird.

4. PD-0184264 zur Verwendung nach einem der Ansprüche 1 bis 3, wobei PD-0184264 einem menschlichen Subjekt an 1 bis 21 aufeinanderfolgenden Tagen, vorzugsweise 5 bis 18 oder 7 bis 14 aufeinanderfolgenden Tagen nach dem stationären Krankenhausaufenthalt, verabreicht wird.

5. PD-0184264 für die Verwendung nach einem der Ansprüche 1 bis 4, wobei PD-0184264 dem menschlichen Subjekt in einer oralen Dosierungsform verabreicht wird.

6. PD-0184264 zur Verwendung nach einem der Ansprüche 1 bis 5, wobei das Coronavirus resistent gegen eine vorherige antivirale Behandlung ist, wobei die vorherige antivirale Behandlung vorzugsweise die Verabreichung von Remdesivir ist.

7. PD-0184264 zur Verwendung nach einem der Ansprüche 1 bis 6, wobei das menschliche Subjekt, das ins Krankenhaus eingeliefert wird, über 60 Jahre alt ist oder zu einer sehr hohen Risikogruppe oder einer Hochrisikogruppe für das Coronavirus gehört.

8. PD-0184264 oder pharmazeutisch akzeptables Salz davon zur Verwendung bei der Behandlung oder Vorbeugung eines COVID-19-Cytokinsturms bei einem mit einem menschlichen Coronavirus infizierten Subjekt.

9. PD-0184264 für die Verwendung nach Anspruch 8, wobei die Verwendung das Reduzieren des Levels von IL-1β und/oder TNF-α in dem Subjekt umfasst, vorzugsweise das Reduzieren des Levels von einem von mehreren, zwei oder mehr, drei oder mehr, vier oder mehr, fünf oder mehr oder allen sechs von TNF-α, IL-1β, IP-10, IL-8, MCP-1 und MIP-1β in einem Subjekt.

10. Pharmazeutische Zusammensetzung, umfassend PD-0184264 oder ein pharmazeutisch akzeptables Salz davon zur Verwendung nach einem der Ansprüche 1 bis 9.

## Revendications

1. PD-0184264 ou un sel pharmaceutiquement acceptable de celui-ci pour une utilisation dans une méthode de traitement d'une maladie causée par un coronavirus chez un sujet humain, dans laquelle le sujet humain est hospitalisé, dans lequel le coronavirus est le SARS-CoV-2 et le patient souffre de COVID-19.

2. PD-0184264 pour l'utilisation de la revendication 1, dans laquelle le COVID-19 est le stade Il du COVID-19.

3. PD-0184264 pour l'utilisation de la revendication 1 ou 2, dans laquelle le PD-0184264 ou son sel pharmaceutiquement acceptable est administré à un sujet humain une fois par jour à une dose de 100 à 1000 mg, de préférence de 300 à 900 mg, de préférence 300, 600 ou 900 mg.

4. PD-0184264 pour l'utilisation de l'une quelconque des revendications 1 à 3, dans laquelle PD-0184264 est administré à un sujet humain pendant 1 à 21 jours consécutifs, de préférence de 5 à 18 jours ou de 7 à 14 jours consécutifs après l'hospitalisation.

5. PD-0184264 pour l'utilisation de l'une quelconque des revendications 1 à 4, dans laquelle PD-0184264 est administré au sujet humain sous une forme posologique orale.

6. PD-0184264 pour l'utilisation de l'une quelconque des revendications 1 à 5, dans laquelle le coronavirus est résistant à un traitement antiviral antérieur, dans lequel le traitement antiviral antérieur est de préférence l'administration de Remdesivir.

7. PD-0184264 pour l'utilisation de l'une quelconque des revendications 1 à 6, dans laquelle le sujet humain hospitalisé est âgé de plus de 60 ans ou appartient à un groupe à risque très élevé ou à un groupe à haut risque pour le coronavirus.

8. PD-0184264 ou sel pharmaceutiquement acceptable pour une utilisation dans le traitement ou la prévention d'une tempête de cytokines COVID-19 chez un sujet infecté par un coronavirus humain.

9. PD-0184264 pour l'utilisation selon la revendication 8, dans laquelle l'utilisation consiste à réduire le niveau d'IL-1β et/ou de TNF-α chez le sujet, de préférence en réduisant le niveau d'un de plus, de deux ou plus, de trois ou plus, de quatre ou plus, de cinq ou plus ou de l'ensemble des six TNF-α, IL-1ß, IP-10, IL-8, MCP-1 et MIP-1β chez un sujet.

10. Composition pharmaceutique comprenant le PD-0184264 ou un sel pharmaceutiquement acceptable de celui-ci pour l'utilisation de l'une quelconque des revendications 1 à 9.
